# FASCICULE DE BREVET EUROPEEN

(11) **EP 2 391 308 B1**
(45) Date de publication et mention de la délivrance du brevet: **23.12.2015**
(21) Numéro de dépôt: 10701363.3
(22) Date de dépôt: 26.01.2010
(51) Int. Cl.: A61F 2/30, A61F 2/44, A61B 17/86, A61B 17/17, A61F 2/46, A61B 17/88

(54) **OUTIL D'IMPLANTATION ET DE FIXATION D'UNE CAGE INTERVERTÉBRALE**
WERKZEUG ZUR IMPLANTATION UND BEFESTIGUNG EINES BANDSCHEIBENGERÜSTS
TOOL FOR IMPLANTING AND ATTACHING AN INTERVERTEBRAL FRAME

(30) Priorité: 27.01.2009 FR 0950490
(43) Date de publication de la demande: 07.12.2011
(73) Titulaire: Acmel Industries, 91160 Champlan (FR)
(72) Inventeur: SOMME, Jean-François, F-92370 Chaville (FR)
(74) Mandataire: Chaffraix, Jean
(86) Numéro de dépôt international: PCT/EP2010/050867
(87) Numéro de publication internationale: WO 2010/086306

(56) Documents cités:
- WO-A-01/62191
- WO-A-2008/042303
- FR-A- 2 795 945
- US-A- 5 397 364

## Description

La présente invention coucerne un outil d'implantation et de fixation d'une cage intervertébrale et plus précisément d'une cage ou cale intervertébrale présentant des vis de fixation dans la vertèbre supérieure et la vertèbre inférieure.

Une cage ou cale intervertébrale est une prothèse mise en place entre deux vertèbres à la place d'un disque endommagé qui a été retiré. Ces cages sont ainsi insérées entre deux vertèbres qu'elles maintiennent écartées l'une de l'autre. De nombreuses formes de cages ont été proposées jusqu'à présent. Ainsi, certaines cages présentent des faces pourvues de moyens d'ancrage de sorte que leur insertion entre les vertèbres est possible mais pas leur retrait (cf. par exemple FR 2 715 293, FR 2 764 795, WO 2004/000176). On a proposé également de réaliser l'ancrage des cages intervertébrales à l'aide de moyens de fixation tels que au moins deux vis implantées pour l'une dans une vertèbre et pour l'autre dans l'autre vertèbre. Une telle cage est notamment décrite dans WO 2008/042303 qui présente une face d'extrémité pourvue de deux alésages filetés obliques à l'intérieur desquels sont vissées deux vis de fixation, la direction oblique des alésages permettant un ancrage dans les vertèbres de part et d'autre de la cage.

Dans EP 1 391 189 est également décrit une cage intervertébrale qui présente un corps principal défini par une paire de surfaces supérieure et inférieure et une paire de surface latérales connectées avec, et des moyens empêchant le retrait qui sont formés sur les surfaces supérieure et/ou inférieure. Cette cage comporte donc des crans d'ancrage sur les faces supérieure et inférieure empêchant son retrait une fois insérée entre deux vertèbres. En outre, la face arrière de corps principal est constituée de deux faces formant un sillon en V. Sur chaque face est prévu un trou eblong pour loger une vis qui traverse le trou pour se loger dans la vertèbre du dessus ou du dessous.

De telles cages pourvues de vis de fixation permettent donc de garantir un encrage sûr et efficace entre deux vertèbres.

Toutefois, la mise en place des moyens de fixation dans les vertèbres n'est pas toujours aisée. En effet, il est important de concilier une bonne implantation et fixation de la cage intervertébrale avec une ouverture limitée. En effet à l'heure actuelle, on cherche à limiter le caractère invasif d'une telle intervention. Il est donc important de pouvoir proposer d'intervenir sur la colonne vertébrale pour implanter une telle prothèse à travers une ouverture réduite. Or la présence de ces moyens de fixation en oblique nécessite l'usage d'un outil de vissage pour les visser au sein de chaque vertèbre selon un angle de vissage peu compatible avec une ouverture réduite pour limiter le caractère invasif.

Dans WO 2008/042303, on a donc proposé un outil permettant l'implantation de la cage décrite. Cet outil est constitué d'un ensemble d'éléments permettant de manipuler un dispositif de vissage qui comprend une poignée, une pince (préhenseur) coopérant avec la poignée et ayant une pluralité de pattes (dents), un guide de vis maintenu en place par les dents pour contrôler la direction des vis perforantes qui sont vissées dans un corps vertébral. Le guide de vis est donc monté à l'extrémité du préhenseur à l'aide des pattes et comporte donc des alésages inclinés en correspondance des alésages de la cage intervertébrale pour mettre en regard un outil de vissage avec la tête de chaque vis. Ce guide permet l'engagement d'un outil de vissage comportant une partie de foret rigide puis une partie flexible et une poignée d'entraînement.

Un tel outil présente un inconvénient important car, l'inclinaison de l'outil de vissage par rapport à l'axe longitudinal du préhenseur portant le guide de vis est de l'ordre de 45° ce qui est encombrant et nécessite une certaine ouverture au niveau du patient. Afin d'éviter en partie cet inconvénient, il est proposé d'utiliser une partie souple entre la partie de foret rigide et la poignée d'entraînement ce qui permet, d'éloigner la zone de manipulation du patient, toutefois on voit mal comment transmettre une force suffisante de vissage à une vis de fixation dans une vertèbre à l'aide d'un tel bras flexible.

Par conséquent, les cages intervertébrales avec lesquelles on propose de réaliser la fixation sur les vertèbres inférieure et supérieure, par l'intermédiaire de vis de fixation inclinées nécessitent toujours une ouverture suffisante du patient pour permettre le vissage du fait de l'angle d'inclinaison des vis de fixation, devant être d'environ 45° par rapport à l'axe l'implantation de la cage. Or on souhaite pouvoir utiliser des cages intervertébrales de ce type, tout en limitant le caractère invasif de l'intervention, en particulier à l'aide d'une ouverture réduite.

La présente invention a donc pour but de proposer un outil d'implantation et de pose d'une cage intervertébrale de ce type qui permet un positionnement sûr est aisé entre deux vertébres ainsi qu'un vissage d'au moins une vis dans au moins l'une des vertèbres entre lesquelles est implantée la cage tout en étant d'une compacité telle que l'encombrement de cet outil en largeur et en épaisseur est limité sensiblement aux dimensions de la cage intervertébrale.

A cet effet l'invention concerne un outil d'implantation et de fixation pour une cage intervertébrale destinée à être insérée entre deux vertèbres, du type pourvu d'au moins un moyen d'ancrage s'étendant de manière oblique dans un alésage de la cage, pour un ancrage dans au moins l'une desdites vertèbres,
ledit outil comportant un axe central longitudinal dont une extrémité est pourvue de moyens de fixation de la cage intervertébrale,
caractérisé en ce que ledit outil comprend en outre un boîtier présentant une extrémité de fixation sur la cage intervertébrale et une extrémité opposée dite de manipulation, boîtier dans lequel sont définis :
un alésage central traversant dans lequel s'étend l'axe central, celui-ci débouchant d'une part de l'extrémité de fixation du boîtier et d'autre part de son extrémité de manipulation,
an moins un canal s'étendant de manière oblique sensiblement depuis une face latérale du boîtier jusqu'à son extrémité de fixation et servant à la réception dudit au moins un moyen d'ancrage de la cage, pour l'amener en regard de l'alésage de la cage,
ainsi qu'au moins un compartiment s'étendant depuis l'extrémité de manipulation du boîtier et débouchant dans le canal pour permettre le guidage d'un outil de vissage depuis l'extrémité de manipulation du boîtier jusqu'à la tête d'un moyen d'ancrage logé dans le canal.

Ainsi, de manière avantageuse, l'ensemble des organes servant à la pose et à la fixation de la cage intervertébrale sont logés an sein de ce boîtier et ce, de manière compacte, entre l'extrémité de fixation et l'extrémité de manipulation du boîtier de sorte que l'encombrement nécessaire à la pose et à la fixation d'une cage de ce type s'éffectue dans un encombrement relativement restreint correspondant sensiblement aux dimensions du boîtier, de manière fiable et sécurisée par rapport au malade et permettant ainsi de limiter l'ouverture à pratiquer pour l'intervention. Le boîtier selon l'invention permet d'amener l'outil de vissage depuis sa face de manipulation jusqu'au moyen d'ancrage de manière sûre et guidée dans le compartiment prévu dans ledit boîtier. Le vissage du moyen d'ancrage s'effectue donc de manière efficace et protégée dans l'espace délimité par le boîtier, limitant ainsi l'ouverture an niveau du patient aux dimensions du boîtier, la manipulation s'effectuant de manière écartée de la cage et du site d'implantation. Par ailleurs les risques de blessures pouvant être liés à cette manipulation de l'outil de visage sont avantageusement éliminés puisque les mouvements intempestifs pouvant survenir lors du vissage sont contenus dans le boîtier.

De préférence, un boîtier pour un outil selon l'invention présente une hauteur minimale d'environ 10 cm.

De manière avantageuse, l'invention au également pour objet un ensemble constitué d'une cage intervertébrale destinée à être insérée entre deux vertèbres, du type pourvu d'au moins un moyen d'ancrage s'étendant de manière oblique dans une alésage de la cage, pour un ancrage dans au moins l'une desdites vertébres, et d'un outil d'implantation et de fixation de ladite cage.

Selon une forme de réalisation préférée, l'outil selon l'invention permet d'implanter une cage intervertébrale pourvue de deux vis de fixation.

De manière préférée, l'extrémité de fixation du boîtier présente des dimensions similaires à celles de la cage de sorte à s'étendre sensiblement dans le prolongement de ladite cage puis le boîtier présente ensuite une partie évasée accueillant en grande partie les canaux de guidage des moyens d'ancrage et formant un épaulement avec la partie d'extrémité de fixation avant de se prolonger de manière sensiblement droite.

De cette manière, l'outil peut avantageusement accompagner la cage jusqu'entre les vertèbres, l'épaulement formé permettant en outre de définir un repère de butée sur les vertèbres correspondant à une mise en place correcte de la cage. La largeur du boîtier après l'épaulement correspondant à l'espace entre les vertèbres et amenant l'extrémité de manipulation du boîtier de manière compacte jusqu'en dehors de la colonne où l'utilisateur peut manipuler aisément les organes de vissage et de fixation dans un encombrement limité au travers d'une ouverture réduite.

De manière avantageuse, les canaux sont ménagés de manière oblique dans le boîtier au niveau de l'extrémité de fixation de celui-ci de manière à amener et guider les moyens d'ancrage en regard des alésages ménagés dans la cage intervertébrale.

L'axe central permet la fixation de la cage intervertébrale à l'extrémité de fixation du boîtier, ledit axe comportant de préférence une extrémité filetée coopérant avec un taraudage ménagé sur la cage en correspondance, l'extrémité opposée de l'axe central en saillie du boîtier à son extrémité de manipulation étant alors pourvue d'un moyen de préhension permettant à l'utilisateur de réaliser la fixation par simple vissage.

Selon une forme de réalisation preférée de l'invention, l'extrémité de fixation du boîtier est réalisée de forme complémentaire à l'extrémité de la cage intervertébrale de manière à favoriser la mise en place de l'outil sur la cage, par coopération de forme desdites extrémités, l'une comportant par exemple une rainure et l'autre une languette correspondante, l'une présentant une ou plusieurs cavités et l'autre des plots logeables dans lesdites cavités, ou toutes autres formes possibles.

Ainsi, de manière préférée, la cage intervertébrale présente une face de fixation constituée d'une rainure en V, un alésage ménagé dans la cage débouchant sur chaque face de la rainure en V pour recevoir les moyens d'ancrage tels que des vis de fixation perforantes, ou tout autre type de vis de fixation approprié, l'extrémité du boîtier étant conformée en forme de V pour se loger dans la rainure, chaque canal de guidage de moyens d'ancrage débauchant en regard des alésages de la cage intervertébrale.

Ainsi, non seulement, on fixe la cage à l'aide des moyens de fixation tels qu'un orifice fileté et l'axe central de l'outil selon l'invention mais en outre, la complémentarité de forme de la cage et du boîtier permet une mise en place aisée de l'outil de pose sur la cage avant pour fixation.

On décrira maintenant l'invention plus est détail on référence au dessin dans lequel :
La figure 1 représente une vue en perspective éclatée d'un outil selon un premier mode de réalisation de l'invention ;
La figure 2 représente une vue de côté de l'outil de la figure 1
La figue 3 représente une vue en coupe selon AA de l'outil de la figure 2 ;
La figure 4 représente une vue en coupe selon BB de l'outil de la figure 2 ;

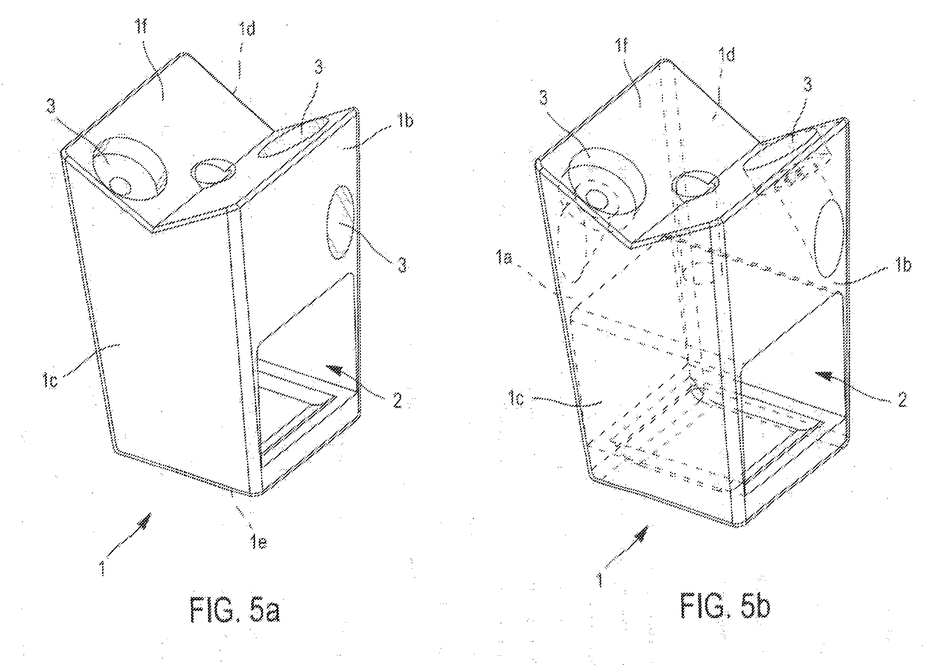

Les figures 5a et 5b représente une vue en perspective latérale d'une cage intervertébrale selon un ensemble selon l'invention;
La figure 6 représente une vue en perspective avant est en transparence d'un second mode de réalisation de l'invention ;

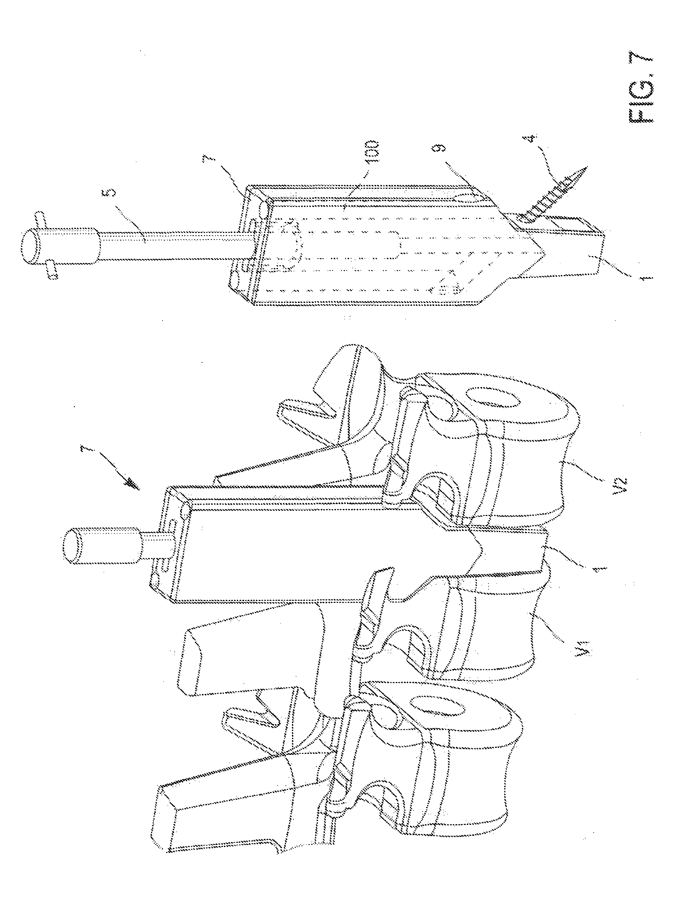

La figure 7 représente une vue en perspective d'un ensemble d'implantation selon l'invention en situation entre deux vertèbres.

L'outil d'implantation et de fixation selon l'invention est destiné à la mise en place d'une cage intervertébrale 1. Cette cage intervertébrale 1 est destinée à être implantée à la place d'un disque entre deux vertèbres V1, V2 et est du type pourvu de moyens d'ancrage tels que des vis de fixation auto taraudeuses ou foreuses s'étendant de manière oblique pour un ancrage dans les plateaux des vertèbres V1, V2 situées de part et d'autre de la cage 1.

De préférence, la cage intervertébrale 1 présente deux faces de contact 1a, 1b opposées destinées à se loger contre les vertèbres V1 et V2, deux faces latérales 1c, 1d et deux faces d'extrémité le et 1f. Un orifice traversant 2 est ménagé dans la cage intervertébrale entre les dieux faces de contact 1a, 1b pour permettre la prolifération osseuse (os spongieux).

L'une des faces d'extrémité 1f dite de fixation se présente en forme de rainure en V, chaque face du V présentant un alésage 3 se prolongeant de manière oblique pour déboucher ensuite sur chaque face de contact 1a, 1b et permettant la mise en place de vis de fixation 4 dans les vertèbres V1, V2.

L'outil selon l'invention comporte un axe central 5 longitudinal dont une extrémité 5a est pourvue de moyens de fixation de la cage intervertébrale 1. Cet axe central 5 présente ainsi une extrémité filetée 5a tandis que la cage intervertébrale 1 présente sur sa face de fixation 1f un taraudage 6 propre à recevoir l'extrémité filetée 5a.

L'outil est constitué d'un boîtier 7 présentant une extrémité de fixation 7a sur la cage intervertébrale 1 et une extrémité opposée dite de manipulation 7b. Ce boîtier 7 selon un premier mode de réalisation est composé de trois parties. Une partie centrale 70 dans laquelle est défini un alésage central traversant 8 dans lequel s'étend l'axe central 5, celui-ci débouchant d'une part de l'extrémité de fixation 7a du boîtier 7 et d'autre part de son extrémité de manipulation 7b.

Sur cette partie centrale 70 est rapportée une premier partie de façade dite avant 71 définissant avec la partie centrale 70 un canal 9 s'étendant respectivement de manière oblique sensiblement depuis une face latérale du boîtier 7 (dans laquelle il est débouchant) jusqu'à son extrémité de fixation 7a et servant à la réception des moyens d'ancrage de la cage 1, à savoir une vis de fixation 4. La vis de fixation 4 peut notamment être introduite par l'extrémité du canal 9 débouchant sur la face latérale du boîtier. Un compartiment 10 s'étend respectivement depuis l'extrémité de manipulation du boîtier 7b et débouche dans le canal 9 pour permettre le guidage d'un organe de vissage 11 jusqu'à la tête 4a de la vis de fixation 4 logée dans le canal 9. De préférence, la tête de la vis de fixation 4 est à six pans creux.

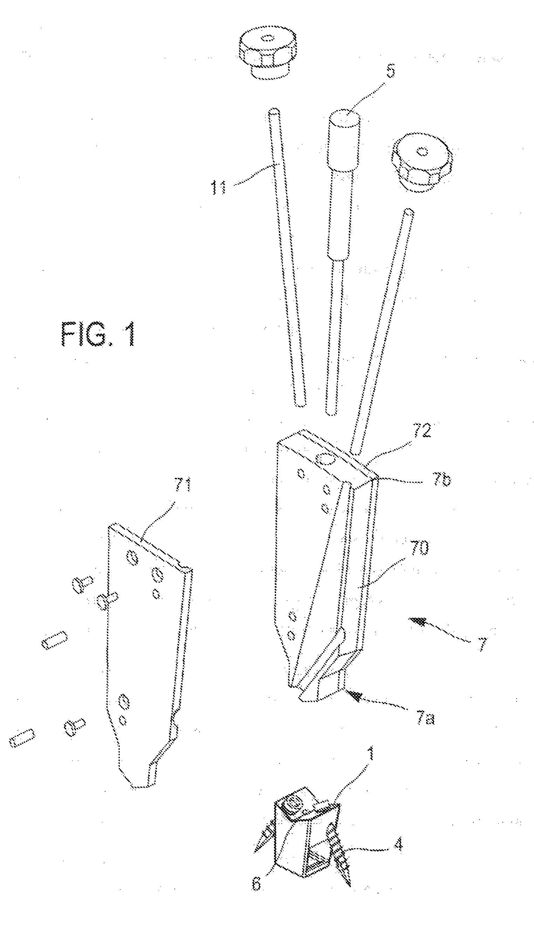

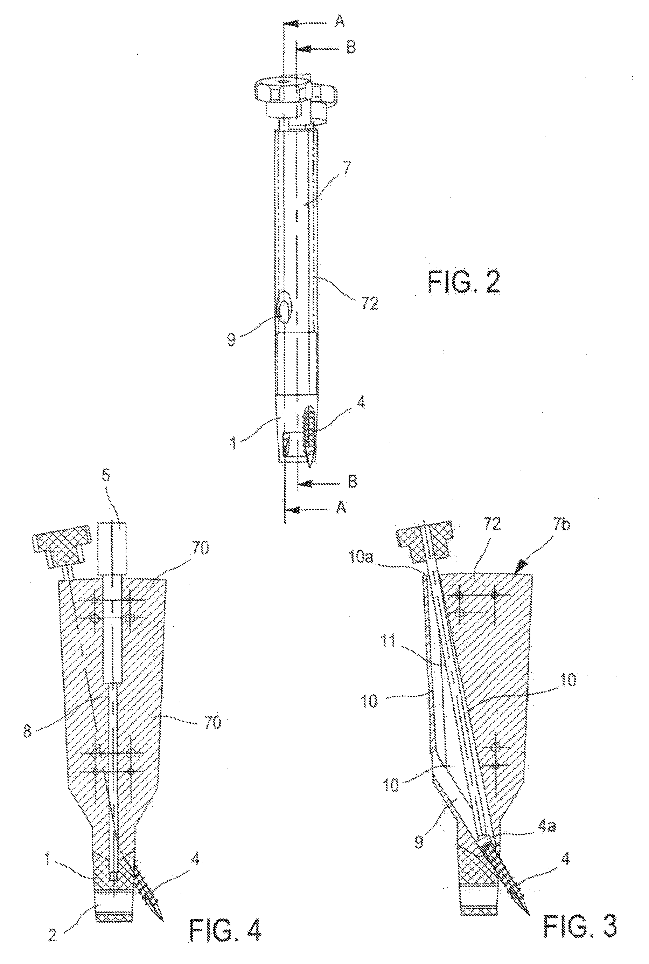

Le canal 9 étant ménagé en oblique et de manière à guider la vis de fixation 4 vers l'orifice 3 ménagé dans la cage 1 jusqu'à son ancrage dans la vertèbre, le compartiment 10 dans le premier mode de réalisation représenté aux figures 1 à 4 présente une forme évasée depuis son extrémité 10a du côté manipulation du boîtier jusqu'à son extrémité opposée qui débouche dans le canal 9, sur tome la longueur dudit canal 9, l'une 10b des parois du compartiment 10 étant parallèle à la paroi latérale du boîtier 7 et l'autre paroi 10c étant inclinée d'un angle d'environ 30° par rapport à ladite paroi 10b. L'extrémité de l'outil de vissage 11 coopère avec la tête de la vis de fixation 4 et celui-ci comportant de préférence une tige rigide est guide dans le compartiment 10 de forme évasée pour suivre la tête de la vis de fixation 4.

Une seconde partie de façade dite arrière 72 est réalisée de manière identique pour ménager sur l'autre côté de la partie centrale 70, un canal 9 et un compartiment 10 pour la seconde vis de fixation 4 de la cage intervertébrale 1.

Comme on peut le voir sur les figures, l'extrémité de fixation 7a du boîtier 7 présente une forme en V de sorte à se loger de manière complémentaire dans la rainure en V de la face d'extrémité de la cage 1.

Les canaux 9 débouchent respectivement sur une face du V en regard des orifices 3 correspondant sur les faces de la rainure en V de la cage 1.

De préférence, la partie d'extrémité en V du boîtier 7 présente des dimensions relativement proches de celles de la cage 1 de sorts à s'étendre sensiblement dans le prolongement de ladite cage 1 puis le boîtier 7 présente ensuite une forme qui s'évase formant un épaulement avant de se prolonger de manière continue.

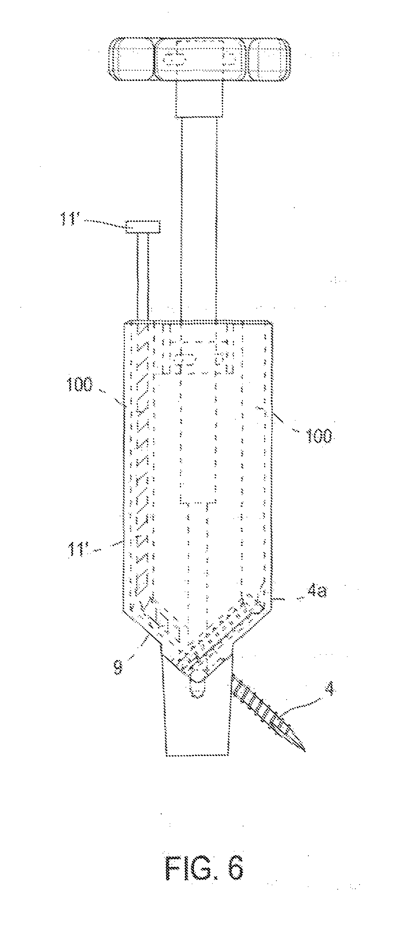

De cette manière, l'outil peux accompagner la cage 1 jusqu'entre les vertèbres V1 et V2 comme on peut le voir sur la figure 6, l'épaulement formé permettant en outre un repère de butée sur les vertèbres V1 et V2 correspondant à une mise en place correcte de la cage 1. La largeur du boîtier 7 après l'épaulement correspondant à l'espace entre les vertèbres V1 et V2 et amenant l'extrémité de manipulation du boîtier de manière compacte jusqu'en dehors de la colonne où l'utilisateur peut manipuler aisément les organes de vissage 11 dans un encombrement limité dans une ouverture réduite. Dans le second mole de réalisation représenté, à la figure 6, le boîtier est réalisé monobloc.

Dans ce boîtier de la figure 6, le compartiment de guidage de l'outil de vissage 11' est formé d'un alésage inversant 100 depuis la face de manipulation 7b du boîtier 7 jusqu'au canal 9, 9' et débouchant dans ledit canal 9 à son extrémité, proche de la face latérale du boîtier 7, (cette extrémité pouvant être débouchante au niveau de ladite face latérale du boîtier). L'outil de vissage 11' comporte une tige déformable tel qu'une tige flexible, pouvant se déformer entre l'alésage 100 et le canal 9 pour suivre la tête de la vis de fixation 4 jusqu'à ce que celle-ci soit correctement ancrée dans la vertèbre.

L'invention n'est bien entendu nullement limitée aux exemples décrits mais englobe toutes les variantes entrant dans la portée de l'invention comme définie dans les revendications.

## Revendications

1. Outil d'implantation et de fixation pour une cage intervertébrale (1) destinée à être insérée entre deux vertèbres (V1, V2), du type pourvu d'au moins un moyen d'ancrage (4) s'étendant de manière oblique dans un alésage (3) de la cage (1), pour un ancrage dans au moins l'une desdites vertèbres (V1, V2),
ledit outil comportant un axe central longitudinal (5) dont une extrémité est pourvue de moyens de fixation de la cage intervertébrale (1),
ledit outil comprenant en outre un boîtier (7) présentant une extrémité de fixation (7a) sur la cage intervertébrale (1) et une extrémité opposée dite de manipulation (7b), boîtier (7) dans lequel sont définis :
un alésage central traversant (8) dans lequel s'étend l'axe central (5), celui-ci débouchant d'une part de l'extrémité de fixation (7a) du boîtier (7) et d'autre part de son extrémité de manipulation (7b),
au moins un canal (9, 9') servant à la réception dudit au moins un moyen d'ancrage (4) de la cage (1), pour l'amener en regard de l'alésage de la cage (1), **caractérisé en ce que** ledit au moins un canal (9,9') s'étend de manière oblique sensiblement depuis une face latérale du boîtier (7) jusqu'à son extrémité de fixation (7a),
et **en ce que** dans le boîtier est également défini au moins un compartiment (10, 100) s'étendant depuis l'extrémité de manipulation (7b) du boîtier (7) et débouchant dans le canal (9, 9') pour permettre le guidage d'un outil de vissage (11) depuis l'extrémité de manipulation (7b) du boîtier (7) jusqu'à la tête d'un moyen d'ancrage (4) logé dans le canal (9, 9').

2. Outil selon la revendication 1,
**caractérisé en ce que** l'extrémité de fixation (7a) du boîtier (7) présente des dimensions similaires à celles de ladite cage (1) de sorte à s'étendre sensiblement dans le prolongement de ladite cage (1) puis le boîtier présente ensuite un partie évasée accueillant en grande partie les canaux de guidage (9, 9') des moyens d'ancrage (4) et formant un épaulement avec la partie d'extrémité de fixation (7a), avant de se prolonger de manière sensiblement droite.

3. Outil selon l'une des revendications 1 et 2,
**caractérisé en ce que** l'extrémité de fixation (7a) du boîtier (7) est réalisée de forme complémentaire à l'extrémité de ladite cage intervertébrale (1) de manière à favoriser la mise en place de l'outil sur ladite cage (1) par coopération de forme desdites extrémités.

4. Outil selon la revendication 3,
**caractérisé en ce que** l'extrémité du boîtier (7a) est conformée en forme de V pour se loger dans une rainure en V ménagée sur la face de fixation (1f) d'une cage (1), chaque canal (9, 9') de guidage de moyens d'ancrage (4) débouchant en regard des alésages (3) de la cage intervertébrale (1).

5. Outil selon l'une des revendications 1 à 4,
**caractérisé en ce que** le canal (9, 9') présente une extrémité débouchant dans une face latérale du boîtier (7).

6. Outil selon l'une des revendications 1 à 5,
**caractérisé en ce que** le compartiment (10) présente une forme évasée depuis son extrémité (10a) du côté manipulation du boîtier, jusqu'à son extrémité qui débouche sur le canal (9) sensiblement sur toute la longueur de celui-ci, l'une (10b) des parois du compartiment (10) étant parallèle à la paroi latérale du boîtier (7) et l'autre paroi (10c) étant inclinée d'un angle d'environ 30° par rapport à ladite paroi (10b).

7. Outil selon l'une des revendications 1 à 5,
**caractérisé en ce que** le compartiment de guidage de l'outil de vissage est formé d'un alésage traversant (100) depuis la face de manipulation (7b) du boîtier (7) jusqu'au canal (9, 9') et débouchant à l'extrémité dudit canal (9, 9') proche de la face latérale du boîtier (7).

8. Outil selon l'une des revendications 1 à 6 comprenant l'outil de vissage (11),
**caractérisé en ce que** l'outil de vissage (11) comporte une tige rigide.

9. Outil selon l'une des revendications 1 à 4 et 7 comprenant l'outil de vissage (11'),
**caractérisé en ce que** l'outil de vissage (11') comporte une tige déformable telle qu'une tige flexible.

10. Outil selon l'une des revendications 1 à 4, 7 et 9,
**caractérisé en ce que** le canal (9') ne débouche pas au niveau de ladite une face latérale du boîtier (7).

11. Outil selon l'une des revendications 1 à 10,
**caractérisé en ce que** le boîtier (7) est réalisé d'une seule pièce.

12. Outil selon l'une des revendications 1 à 11,
**caractérisé en ce que** le boîtier est composé de trois parties, une partie centrale (70) dans laquelle est défini un alésage central traversant (8) dans lequel s'étend l'axe central (5), celui-ci débouchant d'une part de l'extrémité de fixation (7a) du boîtier 7 et d'autre part de son extrémité de manipulation (7b), et sur laquelle sont rapportées deux parties de façades dites avant et arrière (71, 72) définissant avec la partie centrale (70), le canal (9) s'étendant respectivement de manière oblique sensiblement depuis une face latérale du boîtier (7) jusqu'à son extrémité de fixation (7a) et servant à la réception et au guidage des moyens d'ancrage à savoir une vis de fixation 4 et le compartiment (10) s'étendant respectivement depuis l'extrémité de manipulation du boîtier (7b) et débouchant dans le canal (9) pour permettre le guidage d'un organe de vissage (11) jusqu'à la tête (4a) de la vis de fixation 4 logée dans le canal (9).

13. Ensemble constitué d'une cage intervertébrale du type pourvu d'au moins un moyen d'ancrage s'étendant de manière oblique dans un alésage de la cage, pour un ancrage dans au moins l'une desdites vertèbres, et d'un outil d'implantation et de fixation de ladite cage,
**caractérisé en ce que** l'outil est un outil selon l'une des revendications 1 à 12.

14. Ensemble selon la revendication 13,
**caractérisé en ce que** l'extrémité de la cage intervertébrale (1) est réalisée de forme complémentaire à l'extrémité de fixation (7a) du boîtier (7) de manière à favoriser la mise en place de l'outil sur la cage (1) par coopération de forme desdites extrémités.

15. Ensemble selon la revendication 14,
**caractérisé en ce que** la cage intervertébrale (1) présente une face de fixation (1f) constituée d'une rainure en V, au moins un alésage (3) ménagé dans la cage (1) débouchant sur au moins une face de la rainure en V pour recevoir un moyens d'ancrage (4) tels que des vis de fixation perforantes, l'extrémité du boîtier étant conformée en forme de V pour se loger dans la rainure, au moins un canal de guidage (9, 9') de moyens d'ancrage (4) débouchant en regard dudit au moins un alésage (3) de la cage intervertébrale (1).

## Patentansprüche

1. Werkzeug zur Implantation und Befestigung eines Bandscheibengerüsts (1), das zum Einfügen zwischen zwei Wirbeln (V1, V2) von dem Typ bestimmt ist der mit wenigstens einem Verankerungsmittel (4) versehen ist, das sich schräg einer Bohrung (3) des Gerüsts (1) erstreckt, für eine Verankerung in wenigstens einem der genannten Wirbel (V1, V2)
wobei das genannte Werkzeug eine zentrale Längsachse (5) umfasst, von der ein Ende mit Befestigungsmitteln des Bandscheibengerüsts (1) versehen ist,
wobei das genannte Werkzeug darüber hinaus ein Gehäuse (7) umfasst, das en Befestigungsende (7a) auf dem Bandscheibengerüst (1) und ein entgegengesetztes, als Handhabungsende (7b) bezeichnetes Ende aufweist,
ein Gehäuse (7), in dem definiert sind:
eine durchgehende zentrale Bohrung (8), in der sich die zentrale Achse (5) erstreckt, wobei diese einerseits aus dem Befestigungsende (7a) des Gehäuses (7) und andererseits aus seinem Handhabungsende (7b) einmündet,
wobei wenigstens ein Kanal (9, 9') zur Aufnahme des genannten wenigstens einen Verankerungsmittels (4) des Gerüsts (1) dient, um es gegenüber der Bohrung des Gerüsts (1) zu verbringen, **dadurch gekennzeichnet, dass** der genannte wenigstens eine Kanal (9, 9') sich schräg, deutlich von einer lateralen Seite des Gehäuses (7) bis zu seinem Befestigungsende (7a) erstreckt,
und dass in dem Gehäuse ebenfalls wenigstens ein Fach (10, 100) definiert ist, das sich vom Handhabungsende (7b) des Gehäuses (7) erstreckt und in den Kanal (9, 9') einmündet, um die Führung eines Verschraubungswerkzeugs (11) von dem Handhabungsende (7b) des Gehäuses (7) bis zum Kopf eines Verankerungsmittels (4) zu erlauben, das in dem Kanal (9, 9') untergebracht ist.

2. Werkzeug gemäß Anspruch 1,
**dadurch gekennzeichnet, dass** das Befestigungsende (7a) des Gehäuses (7) ähnliche Abmessungen aufweist wie die des genannten Gerüsts(1) derart, dass es sich deutlich in der Verlängerung des genannten Gerüsts (1) erstreckt, das Gehäuse dann anschließend einen ausgeweiteten Teil aufweist, der zum großen Teil die Führungskanäle (9, 9') der Verankerungsmittel (4) aufnimmt und einen Ansatz mit dem Befestigungsendteil (7a) bildet, bevor er sich deutlich gerade verlängert.

3. Werkzeug gemäß Anspruch 1 und 2,
**dadurch gekennzeichnet, dass** das Befestigungsende (7a) des Gehäuses (7) in komplementärer Form zum Ende des genannten Bandscheibengerüsts (1) derart realisiert ist, dass das Einsetzen des Werkzeugs in das genannte Gerüst (1) per Formzusammenwirken der genannten Enden begünstigt wird.

4. Werkzeug gemäß Anspruch 3,
**dadurch gekennzeichnet, dass** das Ende des Gehäuses (7a) in V-Form angepasst wird, um in einer V-förmigen Rinne, die auf der Befestigungsseite (1f) eines Gerüsts (1) ausgespart ist, aufgenommen zu werden, wobei jeder Führungskanal (9, 9') von Verankerungsmitteln (4) gegenüber den Bohrungen (3) des Bandscheibengerüsts (1) einmündet.

5. Werkzeug gemäß Anspruch 1 bis 4,
**dadurch gekennzeichnet, dass** der Kanal (9, 9') ein Ende aufweist, das in einer lateralen Seite des Gehäuses (7) einmündet.

6. Werkzeug gemäß Anspruch 1 bis 5,
**dadurch gekennzeichnet, dass** das Fach (10) eine Form aufweist, die von seinem Ende (10a) von der Handhabungsseite des Gehäuses aus bis zu seinem Ende, das deutlich über die gesamte Länge desselben auf den Kanal (9) einmündet, ausgeweitet ist, wobei eine (10b) der Wände des Fachs (10) parallel zur lateralen Wand des Gehäuses (7) ist und die andere Wand (10c) um einen Winkel von rund 30° im Verhältnis zur genannten Wand (10b) geneigt ist.

7. Werkzeug gemäß Anspruch 1 bis 5,
**dadurch gekennzeichnet, dass** das Führungsfach des Verschraubungswerkzeugs aus einer von der Handhabungsseite (7b) des Gehäuses (7) bis zum Kanal (9, 9') und am Ende des genannten Kanals (9, 9') in der Nähe der lateralen Seite des Gehäuses (7) einmündenden durchquerenden Bohrung (100) geformt ist.

8. Werkzeug gemäß Anspruch 1 bis 6, umfassend das Verschraubungswerkzeug (11), **dadurch gekennzeichnet, dass** das Verschraubungswerkzeug (11) einen steifen Stift umfasst.

9. Werkzeug gemäß Anspruch 1 bis 4 und 7, umfassend das Verschraubungswerkzeug (11'), **dadurch gekennzeichnet, dass** das Verschraubungswerkzeug (11') einen verformbaren Stift wie z. B. einen flexiblen Stift umfasst.

10. Werkzeug gemäß Anspruch 1 bis 4, 7 und 9,
**dadurch gekennzeichnet, dass** der Kanal (9') nicht an der einen genannten lateralen Seite des Gehäuses (7) einmündet.

11. Werkzeug gemäß Anspruch 1 bis 10,
**dadurch gekennzeichnet, dass** das Gehäuse (7) aus einem einzigen Stück realisiert ist.

12. Werkzeug gemäß Anspruch 1 bis 11,
**dadurch gekennzeichnet, dass** das Gehäuse sich aus drei Teilen zusammensetzt, einem zentralen Teil (70), in dem eine durchquerende zentrale Bohrung (8) definiert ist, in der sich die zentrale Achse (5) erstreckt, wobei diese einerseits aus dem Befestigungsende (7a) des Gehäuses (7) einmündet und andererseits aus seinem Handhabungsende (7b) und auf dem zwei als vordere und hintere (71, 72) bezeichnete Fassaden übertragen sind, die mit dem zentralen Teil (70) definieren, wobei sich der Kanal (9) jeweils schräg deutlich von einer lateralen Seite des Gehäuses (7) bis zu seinem Befestigungsende (7a) erstreckt und zur Aufnahme und Führung der Verankerungsmittel dient, und zwar einer Befestigungsschraube 4, und das Fach (10) sich jeweils von dem Handhabungsende des Gehäuses (7b) erstreckt und in den Kanal (9) einmündet, um die Führung eines Verschraubungsorgans (11) bis zum Kopf (4a) der Befestigungsschraube 4 zu erlauben, die in dem Kanal (9) untergebracht ist.

13. Gruppe, die aus einem Bandscheibengerüst von dem Typ gebildet ist, der mit wenigstens einem Verankerungsmittel ausgerüstet ist, das sich schräg in einer Bohrung des Käfigs erstreckt, für eine Verankerung in wenigstens einem der genannten Wirbel, und mit einem Werkzeug zur Implantation und Befestigung des genannten Gerüsts,
**dadurch gekennzeichnet, dass** das Werkzeug ein Werkzeug gemäß Anspruch 1 bis 12 ist.

14. Gruppe gemäß Anspruch 13,
**dadurch gekennzeichnet, dass** das Ende des Bandscheibengerüsts (1) in komplementärer Form zum Befestigungsende (7a) des Gehäuses (7) derart realisiert ist, dass das Einsetzen des Werkzeugs auf dem Gerüst (1) per Formzusammenwirken der genannten Enden begünstigt wird.

15. Gruppe gemäß Anspruch 14,
**dadurch gekennzeichnet, dass** das Bandscheibengerüst (1) eine Befestigungsseite (1f) aufweist, die aus einer V-förmigen Rinne gebildet ist, wobei wenigstens eine Bohrung (3), die in dem Gerüst (1) ausgespart ist, auf wenigstens einer Seite der V-förmigen Rinne einmündet, um ein Verankerungsmittel (4) aufzunehmen, wie z. B. leistungsstarke Befestigungsschrauben, wobei das Ende des Gehäuses V-förmig angepasst ist, um in der Rinne aufgenommen zu werden, wobei wenigstens ein Führungskanal (9, 9') von Verankerungsmitteln (4) gegenüber der genannten wenigstens einen Bohrung (3) des Bandscheibengerüsts (1) einmündet.

## Claims

1. An implantation and fixing tool for a intervertebral cage (1) intended to be inserted between two vertebrae (V1, V2), of the type provided with at least one anchoring means (4) extending obliquely in a bore (3) of the cage (1), for anchoring in at least one of said vertebrae (V1, V2),
said tool comprising a longitudinal central spindle (5), one end of which is provided with means for fixing the intervertebral cage (1),
said tool further comprising a housing (7) having an end (7a) for fixing on the intervertebral cage (1) and an opposite so-called manipulation end (7b),
a housing (6) in which there are defined:
a through central bore (8) in which the central spindle (5) extends, this emerging firstly from the fixing end (7a) of the housing (7) and secondly from its manipulation end (7b),
at least one channel (9, 9') serving for reception of said at least one means (4) for anchoring the cage (1), in order to bring it front of the bore of the cage (1), **characterised in that** said at least one channel (9, 9') extends obliquely substantially from a lateral face of the housing (7) as far as its fixing end (7a),
and **in that** at least one compartment (10, 100) is also defined in the housing, extending from the manipulation end (7b) of the housing (7) and emerging in the channel (9, 9') to allow guidance of a screwing tool (11) from the manipulation end (7b) of the housing (7) as far as the head of an anchoring means (4) housed in the channel (9, 9').

2. A tool according to claim 1,
**characterised in that** the fixing end (7a) of the housing (7) has dimensions similar to those of said cage (1) so as to extend substantially in line with said cage (1), and then the housing next has a splayed part accepting to a major extent the channels (9, 9') guiding the anchoring means (4) and forming a shoulder with the end fixing part (7a), before extending in a substantially straight manner.

3. A tool according to one of claims 1 and 2,
**characterised in that** the fixing end (7a) of the housing (7) is produced in a form complementary to the end of said intervertebral cage (1) so as to assist the placing of the tool on said cage (1) by cooperation of shapes of said ends.

4. A tool according to claim 3,
**characterised in that** the end of the housing (7a) is in a V shape in order to be housed in a V-shaped groove provided on the fixing face (1f) of a cage (1), each channel (9, 9') guiding anchoring means (4) emerging in front of the bores (3) of the intervertebral cage (1).

5. A tool according to one of claims 1 to 4,
**characterised in that** the channel (9, 9') has an end emerging in a lateral face of the housing (7).

6. A tool according one of claims 1 to 5,
**characterised in that** the compartment (10) has a shape splayed from its end (10a) on the manipulation side of the housing as far as its end that emerges on the channel (9) substantially over the entire length thereof, one (10b) of the walls of the compartment (10) being parallel to the lateral wall of the housing (7) and the other wall (10c) being inclined by an angle of approximately 30° with respect to said wall (10b).

7. A tool according one of claims 1 to 5,
**characterised in that** the compartment guiding the screwing tool is formed by a through bore (100) from the manipulation face (7b) of the housing (7) as far as the channel (9, 9') and emerging at the end of said channel (9, 9') close to the lateral face of the housing (7).

8. A tool according one of claims 1 to 6, comprising the screwing tool (11),
**characterised in that** the screwing tool (11) comprises a rigid rod.

9. A tool according one of claims 1 to 4 and 7, comprising the screwing tool (11'),
**characterised in that** the screwing tool (11') comprises a deformable rod such as a flexible rod.

10. A tool according one of claims 1 to 4, 7 and 9,
**characterised in that** the channel (9') does not emerge at said one lateral face of the housing (7).

11. A tool according one of claims 1 to 10,
**characterised in that** the housing (7) is produced in a single piece.

12. A tool according one of claims 1 to 11,
**characterised in that** the housing is composed of three parts, a central part (70) in which a through central bore (8) is defined in which the central spindle (5) extends, this emerging firstly from the fixing end (7a) of the housing (7) and secondly from its manipulation end (7b), and to which there are attached two façade parts referred to as front and rear (71, 72) defining with the central part (70) the channel (9) extending respectively obliquely substantially from a lateral face of the housing (7) as far as its fixing end (7a) and serving to receive and guide anchoring means, namely a fixing screw (4) and the compartment (10) extending respectively from the manipulation end of the housing (7b) and emerging in the channel (9) to allow guidance a screwing member (11) as far as the head (4a) of the fixing screw (4) housed in the channel.

13. An assembly consisting of an intervertebral cage of the type provided with at least one anchoring means extending obliquely in a bore of the cage, for anchoring in at least one of said vertebrae, and a tool for implanting and fixing said cage,
**characterised in that** the tool is a tool according to one of claims 1 to 12.

14. An assembly according to claim 13,
**characterised in that** the end of the intervertebral cage (1) is produced with a form complementary to the fixing end (7a) of the housing (7) so as to assist the placing of the tool on the cage (1) by cooperation of shapes of said ends.

15. An assembly according to claim 14,
**characterised in that** the intervertebral cage (1) has a fixing face (1f) consisting of a V-shaped groove, at least one bore (3) provided in the cage (1) emerging on at least one face of the V-shaped groove in order to receive an anchoring means (4) such as perforating fixing screws, the end of the housing being conformed in a V in order to house in the groove at least one channel (9, 9') guiding anchoring means (4) emerging in front of said at least one bore (3) of the intervertebral cage (1).
